# EUROPEAN PATENT APPLICATION

(11) **EP 1 306 086 A2**
(43) Date of publication of application: **02.05.2003**
(21) Application number: 03075243.0
(22) Date of filing: 25.04.1994
(51) Int. Cl.: A61K 31/42, A61K 31/425, A61K 31/44, A61K 31/505

(54) **Use of thiazolidinediones for the treatment of atherosclerosis and eating disorders**

(30) Priority: 23.04.1993 GB 9308487
(62) Divisional of application: 94913693.1
(71) Applicant: SMITHKLINE BEECHAM PLC, Brentford, Middlesex TW8 9GS (GB)
(72) Inventor: Cawthorne, Michael Anthony, c/o GlaxosmithKline, Harlow, Essex CM19 5AW (GB); Hindley, Richard Mark, c/o GlaxosmithKline, Harlow, Essex CM19 5AW (GB)
(74) Representative: Rutter, Keith

(57) **Abstract**

The use of a compound of formula (I): 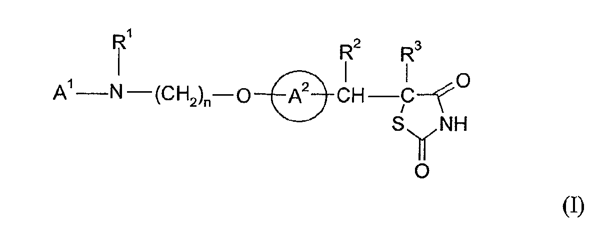 or a tautomeric form thereof and/or a pharmaceutically acceptable salt thereof and/or a pharmaceutically acceptable solvate thereof, wherein:
A¹ represents a substituted or unsubstituted aromatic heterocyclyl group;
R¹ represents a hydrogen atom, an alkyl group, an acyl group, an aralkyl group, wherein the aryl moiety may be substituted or unsubstituted, or a substituted or unsubstituted aryl group;
R² and R³ each represent hydrogen, or R² and R³ together represent a bond;
A² represents a benzene ring having in total up to five substituents; and
n represents an integer in the range of from 2 to 6, for the manufacture of a medicament for use in the treatment and/or prophylaxis of atherosclerosis and/or for the regulation of appetite and food intake.

## Description

This invention relates to novel use of certain substituted thiazolidinedione derivatives and of pharmaceutical compositions containing such compounds.

European Patent Applications, Publication Numbers 0008203, 0139421, 0155845, 0177353, 0193256, 0207581 and 0208420 relate to thiazolidinedione derivatives which are disclosed as having hypoglycaemic and hypolipidaemic activity. Chem. Pharm. Bull 30 (10) 3580-3600 also relates to certain thiazolidinedione derivatives having hypoglycaemic and hypolipidaemic activities.

European Patent Application, Publication Number 0306228 discloses certain substituted thiazolidinedione derivatives of formula (A): 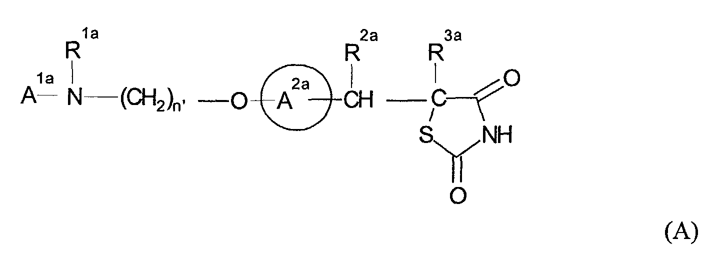 or a tautomeric form thereof and/or a pharmaceutically acceptable salt thereof, and/or a pharmaceutically acceptable solvate thereof, wherein:
A^{1a} represents a substituted or unsubstituted aromatic heterocyclyl group;
R^{1a} represents a hydrogen atom, an alkyl group, an acyl group, an aralkyl group, wherein the aryl moiety may be substituted or unsubstituted, or a substituted or unsubstituted aryl group;
R^{2a} and R^{3a} each represent hydrogen, or R^{2a} and R^{3a} together represent a bond;
A^{2a} represents a benzene ring having in total up to five substituents; and
n' represents an integer in the range of from 2 to 6. Such compounds are disclosed *inter alia* as being useful for the treatment and/or prophylaxis of cardiovascular disease and certain eating disorders.

It has now surprisingly been discovered that these compounds are of particular use in the treatment and/or prophylaxis of atherosclerosis. In addition these compounds are particularly useful for the regulation of appetite and food intake in subjects suffering from disorders associated with under-eating, such as anorexia nervosa, and disorders associated with over-eating, such as obesity and anorexia bulimia.

Accordingly, the present invention provides the use of a compound of formula (I): 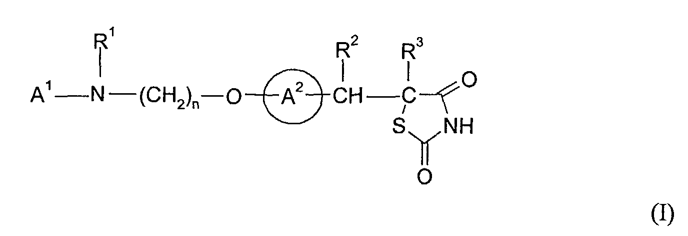 or a tautomeric form thereof and/or a pharmaceutically acceptable salt thereof, and/or a pharmaceutically acceptable solvate thereof, wherein:
A¹ represents a substituted or unsubstituted aromatic heterocyclyl group;
R¹ represents a hydrogen atom, an alkyl group, an acyl group, an aralkyl group, wherein the aryl moiety may be substituted or unsubstituted, or a substituted or unsubstituted aryl group;
R² and R³ each represent hydrogen, or R² and R³ together represent a bond;
A² represents a benzene ring having in total up to five substituents; and n represents an integer in the range of from 2 to 6, for the manufacture of a medicament for treatment and/or prophylaxis of atherosclerosis and/or for the regulation of appetite and food intake in subjects suffering from disorders associated with under-eating ,such as anorexia nervosa, and disorders associated with over-eating, such as obesity and anorexia bulimia.

Suitable aromatic heterocyclyl groups include substituted or unsubstituted, single or fused ring aromatic heterocyclyl groups comprising up to 4 hetero atoms in each ring selected from oxygen, sulphur or nitrogen.

Favoured aromatic heterocyclyl groups include substituted or unsubstituted single ring aromatic heterocyclyl groups having 4 to 7 ring atoms, preferably 5 or 6 ring atoms.

In particular, the aromatic heterocyclyl group comprises 1, 2 or 3 heteroatoms, especially 1 or 2, selected from oxygen, sulphur or nitrogen.

Suitable values for A¹ when it represents a 5- membered aromatic heterocyclyl group include thiazolyl and oxazolyl, especially oxazolyl.

Suitable values for A¹ when it represents a 6- membered aromatic heterocyclyl group include pyridyl or pyrimidinyl.

Suitably R² and R³ each represent hydrogen.

Preferably, A¹ represents a moiety of formula (a), (b) or (c): 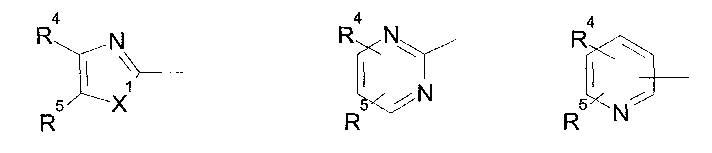 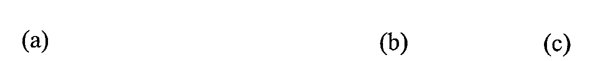 wherein: R⁴ and R⁵ each independently represents a hydrogen atom, an alkyl group or a substituted or unsubstituted aryl group or when R⁴ and R⁵ are each attached to adjacent carbon atoms, then R⁴ and R⁵ together with the carbon atoms to which they are attached form a benzene ring wherein each carbon atom represented by R⁴ and R⁵ together may be substituted or unsubstituted; and in the moiety of formula (a)
X represents oxygen or sulphur.

Aptly, A¹ represents a moiety of the abovedefined formula (a).

Aptly, A¹ represents a moiety of the abovedefined formula (b).

Aptly, A¹ represents a moiety of the abovedefined formula (c).

In one favoured aspect R⁴ and R⁵ together represent a moiety of formula (d): 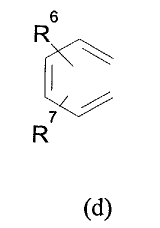 wherein R⁶ and R⁷ each independently represent hydrogen, halogen, substituted or unsubstituted alkyl or alkoxy.

Suitably, R⁶ and R⁷ each independently represent hydrogen, halogen, alkyl or alkoxy.

Favourably, R⁶ represents hydrogen. Favourably, R⁷ represents hydrogen.

Preferably, R⁶ and R⁷ both represent hydrogen.

In a further favoured aspect R⁴ and R⁵ each independently represent hydrogen, alkyl or a substituted or unsubstituted phenyl group and more favourably, R4 and R5 each independently represent hydrogen, alkyl or phenyl.

Preferably, for the moiety of formula (a), R⁴ and R⁵ together represent the moiety of formula (d).

Preferably, for the moieties of formula (b) or (c), R⁴ and R⁵ both represent hydrogen.

It will be appreciated that the five substituents of A² include three optional substituents. Suitable optional substituents for the moiety A² include halogen, substituted or unsubstituted alkyl or alkoxy.

Favourably, A² represents a moiety of formula (e): 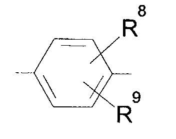 wherein R⁸ and R⁹ each independently represent hydrogen, halogen, substituted or unsubstituted alkyl or alkoxy.

Suitably, R⁸ and R⁹ each independently represent hydrogen, halogen, alkyl or alkoxy. Preferably, R⁸ and R⁹ each represent hydrogen.

Favourably, X represents oxygen. Favourably, X represents sulphur.

In one preferred aspect the present invention provides a class of compounds, which fall wholly within the scope of formula (I), of formula (II): 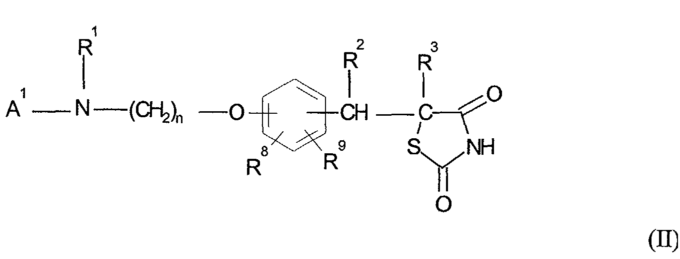 or a tautomeric form thereof, and/or a pharmaceutically acceptable salt thereof and/or a pharmaceutically acceptable solvate thereof, wherein A¹, R¹, R², R³, and n are as defined in relation to formula (I) and R⁸ and R⁹ are as defined in relation to formula (e).

Suitably, n represents an integer 2, 3 or 4, notably 2 or 3 and especially 2.

Suitably, R¹ represents hydrogen, alkyl, acyl, especially acetyl, or benzyl.

When R¹ represents an alkyl group, examples of such alkyl groups include methyl and isopropyl. Preferably, R¹ represents a methyl group.

As indicated above a compound of formula (I) may exist in one of several tautomeric forms, all of which are encompassed by the present invention. It will be appreciated that the present invention encompasses all of the isomeric forms of the compounds of formula (I) and the pharmaceutically acceptable salts thereof, including any stereoisomeric forms thereof, whether as individual isomers or as mixtures of isomers.

Suitable substituents for any heterocyclyl group include up to 4 substituents selected from the group consisting of: alkyl, alkoxy, aryl and halogen or any two substituents on adjacent carbon atoms, together with the carbon atoms to which they are attached, may form an aryl group, preferably a benzene ring, and wherein the carbon atoms of the aryl group represented by the said two substituents may themselves be substituted or unsubstituted.

When used herein the term 'aryl' includes phenyl and naphthyl optionally substituted with up to five, preferably up to three, groups selected from halogen, alkyl, phenyl, alkoxy, haloalkyl, hydroxy, amino, nitro, carboxy, alkoxycarbonyl, alkoxycarbonylalkyl, alkylcarbonyloxy, or alkylcarbonyl groups.

When used herein the term 'halogen' refers to fluorine, chlorine, bromine and iodine; preferably chlorine.

When used herein the terms 'alkyl' and 'alkoxy' relate to groups having straight or branched carbon chains,containing up to 12 carbon atoms.

When used herein the term 'acyl' includes alkylcarbonyl groups.

Suitable alkyl groups are C₁₋₁₂ alkyl groups, especially C₁₋₆ alkyl groups e.g. methyl, ethyl, n-propyl, iso-propyl, n-butyl, isobutyl or tert-butyl groups.

Suitable substituents for any alkyl group include those indicated above in relation to the term "aryl".
Suitable pharmaceutically acceptable salts include salts of the thiazolidinedione moiety, and, where appropriate, salts of carboxy groups.

Suitable pharmaceutically acceptable salts of the thiazolidinedione moiety include metal salts especially alkali metal salts such as the lithium, sodium and potassium salts.

Suitable pharmaceutically acceptable salts of carboxy groups include metal salts, such as for example aluminium, alkali metal salts such as sodium or potassium, alkaline earth metal salts such as calcium or magnesium and ammonium or substituted ammonium salts, for example those with lower alkylamines such as triethylamine, hydroxy alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-b-phenethylamine, dehydroabietylamine, N,N'-bisdehydroabietylamine, glucamine, N-methylglucamine or bases of the pyridine type such as pyridine, collidine or quinoline.

Suitable pharmaceutically acceptable solvates include hydrates.

The salts and/or solvates of the compounds of formula (I) may be prepared and isolated according to conventional procedures for example sodium salts may be prepared by using sodium methoxide in methanol.

Suitable pharmaceutically acceptable salts of the thiazolidinedione moiety include metal salts especially alkali metal salts such as the lithium, sodium and potassium salts.

A preferred compound of formula (I) is 5-[4-[2-(N-methyl-N-(2-pyridyl)amino)ethoxy]benzyl]thiazolidine-2,4-dione or a tautomeric form thereof and/or a pharmaceutically acceptable salt thereof and/or a pharmaceutically acceptable solvate thereof.

A compound of formula (I), or the tautomeric form thereof, and/or a pharmaceutically acceptable salt thereof, and/or a pharmaceutically acceptable solvate thereof, may be prepared using the processes described in EP 0306228. The contents of EP 0306228 are incorporated herein by reference

As mentioned above the compounds of the invention are indicated as having useful therapeutic properties:

A compound of formula (I), or a tautomeric form thereof and/or a pharmaceutically acceptable salt thereof and/or a pharmaceutically acceptable solvate thereof, may be administered per se or, preferably, as a pharmaceutical composition also comprising a pharmaceutically acceptable carrier.

As used herein the term 'pharmaceutically acceptable' embraces compounds, compositions and ingredients for both human and veterinary use: for example the term 'pharmaceutically acceptable salt' embraces a veterinarily acceptable salt.

The composition may, if desired, be in the form of a pack accompanied by written or printed instructions for use.

Usually the pharmaceutical compositions of the present invention will be adapted for oral administration, although compositions for administration by other routes, such as by injection and percutaneous absorption are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Most suitably the composition will be formulated in unit dose form. Such unit dose will normally contain an amount of the active ingredient in the range of from 0.1 to 1000 mg, more usually 0.1 to 500 mg, and more especially 0.1 to 250 mg.

The present invention further provides a method for the treatment of atherosclerosis, in a human or non-human mammal, which comprises administering an effective, non-toxic, amount of a compound of formula (I), or a tautomeric form thereof and/or a pharmaceutically acceptable salt thereof and/or a pharmaceutically acceptable solvate thereof, to a human or non-human mammal in need thereof.

The present invention also provides a method for the regulation of appetite and food intake in disorders associated with under-eating, such as anorexia nervosa, and disorders associated with over-eating, such as obesity and anorexia bulimia, in a human or non-human mammal, which comprises administering an effective, non-toxic, amount of a compound of formula (I), or a tautomeric form thereof and/or a pharmaceutically acceptable salt thereof and/or a pharmaceutically acceptable solvate thereof, to a human or non-human mammal in need thereof.

Conveniently, the active ingredient may be administered as a pharmaceutical composition hereinbefore defined, and this forms a particular aspect of the present invention.

In the above mentioned treatments the compound of the general formula (I), or a tautomeric form thereof and/or a pharmaceutically acceptable salt thereof and/or a pharmaceutically acceptable solvate thereof, may be taken in doses, such as those described above, one to six times a day in a manner such that the total daily dose for a 70 kg adult will generally be in the range of from 0.1 to 6000 mg, and more usually about 1 to 1500 mg.

In the treatment and/or prophylaxis of non-human mammals, especially dogs, the active ingredient may be adminstered by mouth, usually once or twice a day and in an amount in the range of from about 0.025 mg/kg to 25 mg/kg, for example 0.1 mg/kg to 20 mg/kg.

The therapeutic activity of a compound of formula (I), a tautomeric form thereof, a pharmaceutically acceptable salt thereof or a pharmaceutically acceptable solvate thereof may be demonstrated using conventional test methods, for example anti-atherosclerotic activity may be demonstrated using methods disclosed in Journal of Clinical Investigations 1992, Vol 89, page 706-711.

## Claims

1. The use of a compound of formula (I): 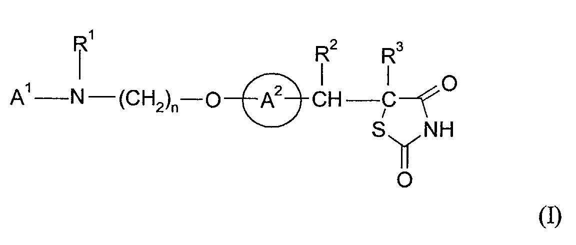 or a tautomeric form thereof and/or a pharmaceutically acceptable salt thereof and/or a pharmaceutically acceptable solvate thereof, wherein:
A¹ represents a substituted or unsubstituted aromatic heterocyclyl group;
R¹ represents a hydrogen atom, an alkyl group, an acyl group, an aralkyl group, wherein the aryl moiety may be substituted or unsubstituted, or a substituted or unsubstituted aryl group;
R² and R³ each represent hydrogen, or R² and R³ together represent a bond;
A² represents a benzene ring having in total up to five substituents; and
n represents an integer in the range of from 2 to 6, for the manufacture of a medicament for use in the treatment and/or prophylaxis of atherosclerosis and/or for the regulation of appetite and food intake.

2. A use according to claim 1 wherein in the compound of formula (I), A¹ represents a substituted or unsubstituted, single or fused ring aromatic heterocyclyl group comprising up to 4 hetero atoms in the ring selected from oxygen, sulphur or nitrogen.

3. A use according to claim 1 or claim 2 wherein in the compound of formula (I), A¹ represents a moiety of formula (a), (b) or (c): 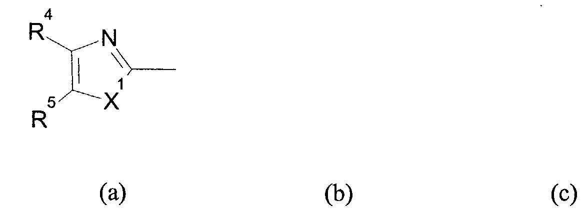 wherein:
R⁴ and R⁵ each independently represents a hydrogen atom, an alkyl group or a substituted or unsubstituted aryl group or when R⁴ and R⁵ are each attached to a carbon atom, then R⁴ and R⁵ together with the carbon atoms to which they are attached form a benzene ring wherein each carbon atom represented by R⁴ and R⁵ together may be substituted or unsubstituted; and in the moiety of formula (a)
X represents oxygen or sulphur.

4. A use according to claim 3, wherein R⁴ and R⁵ each independently represent hydrogen, alkyl or a substituted or unsubstituted phenyl group.

5. A use according to claim3 or claim 4, wherein in the compound of formula (I), R⁴ and R⁵ together represent a moiety of formula (d): 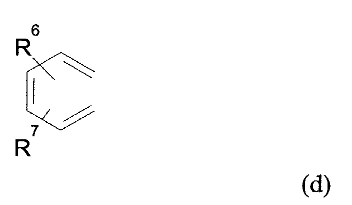 wherein R⁶ and R⁷ each independently represent hydrogen, halogen, substituted or unsubstituted alkyl or alkoxy.

6. A use according to claim 5, wherein in the moiety of formula (d) R⁶ and R⁷ both represent hydrogen.

7. A use according to any one of claims 1 to 6, wherein in the compound of formula
(I) A² represents a moiety of formula (e): 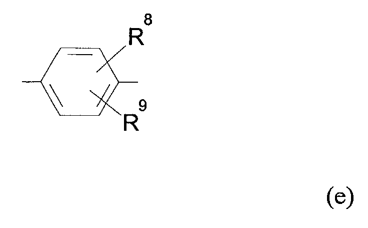
wherein R⁸ and R⁹ each independently represent hydrogen, halogen, substituted or unsubstituted alkyl or alkoxy.

8. A use according to claim 7, wherein in the moiety of formula (e) R⁸ and R⁹ each represent hydrogen.

9. A use according to claim 1, wherein the compound of formula (I) is a compound of formula (II): 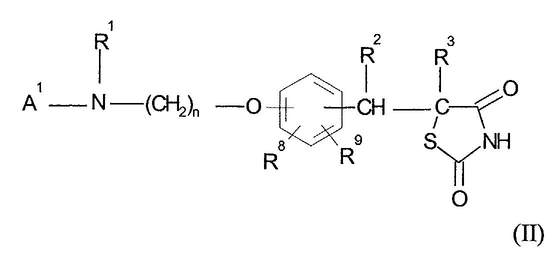 or a tautomeric form thereof and/or a pharmaceutically acceptable salt thereof and/or a pharmaceutically acceptable solvate thereof, wherein A¹, R¹, R², R³ and n are as defined in relation to formula (I) in claim 1 and R⁸ and R⁹ are as defined in relation to formula (e) in claim 7.

10. A use according to any one of claims 1 to 9, wherein in the compound of formula (I) n represents an integer 2 or 3.

11. A use according to any one of claims 1 to 3, wherein in the compound of formula (I) R¹ represents a methyl group.

12. A use according to any one of claims 1 to 3, wherein the compound of formula (I) is selected from the list consisting of:
5-(4-[2-(N-methyl-N-(2-benzothiazolyl)amino)ethoxy]benzyl)-2,4-thiazolidinedione;
5-(4-[2-(N-methyl-N-(2-benzothiazolyl)amino)ethoxy]benzylidene)-2,4-thiazolidinedione;
5-(4-[2-(N-methyl-N-(2-benzoxazolyl)amino)ethoxy] benzyl)-2,4-thiazolidinedione;
5-(4-[2-(N-methyl-N-(2-benzoxazolyl)amino)ethoxy]benzylidene)-2,4-thiazolidinedione;
5-(4-[2-(N-methyl-N-(2-pyrimidinyl)amino)ethoxy]benzyl)-2,4-thiazolidinedione;
5-(4-[2-(N-methyl-N-(2-pyrimidinyl)amino)ethoxy]benzylidene)-2,4-thiazolidinedione;
5-(4-(2-(N-methyl-N-[2-(4,5-dimethylthiazolyl)]amino)ethoxy]benzyl)-2,4-thiazolidinedione;
5-(4-[2-(N-methyl-N-[2-(4,5-dimethylthiazolyl)]amino)ethoxy]benzylidene)-2,4-thiazolidinedione;
5-(4-[2-(N-methyl-N-(2-thiazolyl)amino)ethoxy]benzyl)-2,4-thiazolidinedione;
5-(4-[2-(N-methyl-N-(2-thiazolyl)amino)ethoxy]benzylidene)-2,4-thiazolidinedione;
5-[4-(2-(N-methyl-N-(2-(4-phenylthiazolyl))amino)ethoxy)benzyl]-2,4-thiazolidinedione;
5-(4-[2-(N-methyl-N-(2-(4-phenylthiazolyl))amino)ethoxy]benzylidene)-2,4-thiazolidinedione;
5-(4-[2-(N-methyl-N-[2-(4-phenyl-5-methylthiazolyl)]amino)ethoxy]benzyl)-2,4-thiazolidinedione;
5-(4-[2-(N-methyl-N-[2-(4-phenyl-5-methylthiazolyl)]amino)ethoxy]benzylidene)-2,4 -thiazolidinedione;
5-(4-[2-(N-methyl-N-[2-(4-methyl-5-phenylthiazolyl)]amino)ethoxy]benzyl)-2,4-thiazolidinedione;
5-(4-[2-(N-methyl-N-[2-(4-methyl-5-phenylthiazolyl)]amino)ethoxy]benzylidene)-2,4-thiazolidinedione;
5-(4-[2-(N-methyl-N-[2-(4-methylthiazolyl)]amino)ethoxy]benzyl)-2,4-thiazolidinedione;
5-(4-[2-(N-methyl-N-[2-(4-methylthiazolyl)]amino)ethoxy]benzylidene)-2,4-thiazolidinedione;
5-[4-(2-(N-methyl-N-[2-(5-phenyloxazolyl)]amino)ethoxy)benzyl]-2,4-thiazolidinedione;
5-(4-[2-(N-methyl-N-[2-(5-phenyloxazolyl)]amino)ethoxy]benzylidene)-2,4-thiazolidinedione;
5-(4-[2-(N-methyl-N-[2-(4,5-dimethyloxazolyl)] amino)ethoxy]benzyl)-2,4-thiazolidinedione;
5-(4-[2-(N-methyl-N-[2-(4,5-dimethyloxazolyl)]amino)-ethoxy]benzylidene)-2,4-thiazolidinedione;
5-[4-(2-(2-pyrimidinylamino)ethoxy)benzyl]-2,4-thiazolidinedione;
5-[4-(2-(2-pyrimidinylamino)ethoxy)benzylidene]-2,4-thiazolidinedione;
5-(4-[2-(N-ccetyl-N-(2-pyrimidinyl)amino)ethoxy]benzyl)-2,4-thiazolidinedione;
5-(4-(2-(N-(2-benzothiazolyl)-N-benzylamino)ethoxy)benzylidene)-2,4-thiazolidinedione;
5-(4-(2-(N-(2-benzothiazolyl)-N-benzylamino)ethoxy)benzyl)-2,4-thiazolidinedione;
5-(4-[3-(N-methyl-N-(2-benzoxazolyl)amino)propoxy]benzyl)-2,4-thiazolidinedione;
5-(4-[3-(N-methyl-N-(2-benzoxazolyl)amino)propoxy]benzylidene)-2,4-thiazolidinedione;
5-(4-[2-(N-methyl-N-(2-pyridyl)amino)ethoxy]benzyl)- 2,4-thiazolidinedione; and
5-(4-[2-(N-methyl-N-(2-pyridyl)amino)ethoxy]benzylidene)-2,4-thiazolidinedione; or a tautomeric form thereof and/or a pharmaceutically acceptable salt thereof and/or a pharmaceutically acceptable solvate thereof.

13. A use according to claim 1, wherein the compound of formula (I) is 5-(4-[2-(N-methyl-N-(2-pyridyl)amino)ethoxy]benzyl)- 2,4-thiazolidinedione; or a tautomeric form thereof and/or a pharmaceutically acceptable salt thereof and/or a pharmaceutically acceptable solvate thereof.
